(19) Europäisches Patentamt
European Patent Office
Office européen des brevets

(11) Veröffentlichungsnummer: **0 268 223**
**A2**

(12) # EUROPÄISCHE PATENTANMELDUNG

(21) Anmeldenummer: **87116760.7**

(22) Anmeldetag: **13.11.87**

(51) Int. Cl.⁴: **C07D 498/06** , C07D 513/06 , C07D 471/06 , C07D 455/04 , C07D 215/56 , C07D 471/04 , A61K 31/535 , A61K 31/54 , A61K 31/435 , A61K 31/47 , //(C07D498/06,265:00,221:00)

(30) Priorität: **18.11.86 DE 3639465**

(43) Veröffentlichungstag der Anmeldung: **25.05.88 Patentblatt 88/21**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Duerckheimer, Walter, Dr.**
**Im Lerchenfeld 45**
**D-6234 Hattersheim am Main(DE)**
Erfinder: **Leube, Karl**
**Hostertstrasse 6**
**D-6231 Sulzbach(DE)**

(54) **Optisch aktive Gyrasehemmer, ihre Herstellung und Verwendung als Antibiotika.**

(57) Pharmazeutische wirksame, optisch aktive Antipoden von Verbindungen der Formeln

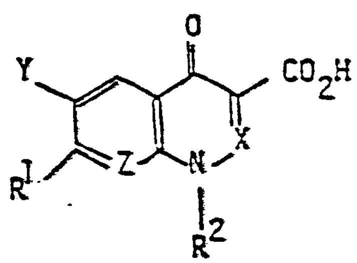

(I) und

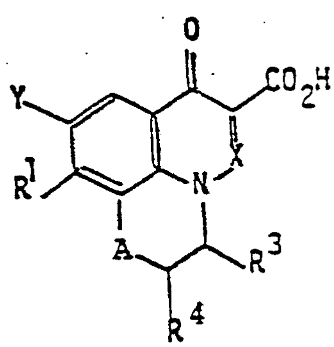

(II)

ein Verfahren zu ihrer Herstellung, sowie pharmazeutische Präparate, die eine solche Verbindung enthalten und die Verwendung der Verbindungen zur Bekämpfung bakterieller Infektionen.

EP 0 268 223 A2

Xerox Copy Centre

**Optisch aktive Gyrasehemmer, ihre Herstellung und Verwendung als Antibiotika**

Gyrasehemmer vom Typ der Nalidixinsäure, des Norfloxacins. Ciprofloxacins. Enoxacins. Pefloxacins und Ofloxacins sind Chinoloncarbonsäuren und haben als Mittel zur Behandlung bakterieller Infektionen große Bedeutung. Auch mit den Chinolonen verwandte Ringsysteme, wie sie in Drugs of the Future. Band 11, Nr. 5, 1986, S. 366 - 369 zusammengefaßt sind, besitzen bei geeigneter Substitution eine antibiotische Wirksamkeit, die den obengenannten Prototypen vergleichbar ist.

Unter den hochwirksamen Gyrasehemmern gibt es Strukturen, die ein Asymmetriezentrum besitzen, d.h., es gibt eine rechtsdrehende und eine linksdrehende Form. Sie werden aber bisher nur in racemischer Form angewendet. Ein Hauptgrund liegt darin, daß diese Wirkstoffe Betaincharakter besitzen, schwer löslich sind und die Trennung in die Antipoden über diastereomere Salze bisher nicht möglich war.

Beispielsweise konnte die (-) und (+) Form von Ofloxacin bisher nur durch eine schwierige, vielstufige Synthese erhalten werden, indem man eine (±) 3,5-Dinitrobenzoyl-Zwischenstufe von Ofloxacin mittels Hochdruckflüssigkeits-Chromatographie (HPLC) auftrennte und die zu diesem Zweck eingeführte Alkoholfunktion in mehreren Syntheseschritten zur Methylgruppe reduzieren mußte (Antimicrobial Ag.& Chemother. 29 (1986), S. 163). Ein solcher Weg zur Herstellung von optisch aktivem Ofloxacin ist technisch nicht durchführbar. Es bestand daher das Bedürfnis, einen gangbaren Weg zur Herstellung optisch aktiver Gyrasehemmer, insbesondere von Ofloxacin zu entwickeln.

Der vorliegenden Erfindung liegt nun die Idee zugrunde, betainartige Gyrasehemmer vom Chinolontyp, die als Racemate vorliegen, über Hydrazinium-Zwischenstufen mit Hilfe optisch aktiver Säuren in deren Antipoden zu spalten und daraus anschließend die optisch aktiven Wirkstoffe freizusetzen.

Gegenstand der Erfindung ist somit ein Verfahren zur Herstellung der optisch aktiven Antipoden von Gyrasehemmern der allgemeinen Formeln I und II

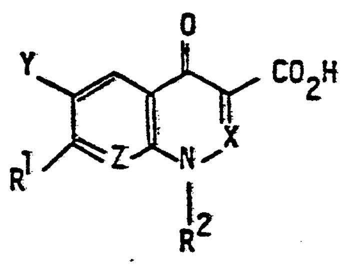

(I)

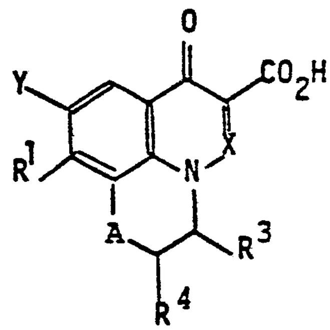

(II)

in denen bedeuten

X = -CH=, -N= oder =CF-

Y = Wasserstoff oder Halogen,

Z = -CH=, -N=, =CF-oder

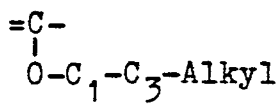

A = $-CH_2-$, -O-oder -S-

$R^1$ = $C_1$-$C_4$-Alkyl, das durch Dimethylamino oder Diethylamino substituiert ist, gesättigter oder ungesättigter 5-oder 6-gliedriger Ring mit 1 bis 2 Stickstoffatomen, der auch weiter substituiert sein kann durch $C_1$-$C_4$-Alkyl, Hydroxy-$C_1$-$C_4$-alkyl oder $C_1$-$C_4$-Dialkylamino,

$R^2$ = $C_1$-$C_4$-Alkyl, $C_2$-$C_3$-Alkenyl oder Cyclopropy, wobei diese Reste auch substituiert sein können durch Fluor, Chlor, Hydroxy, Methoxy, Ethoxy oder Diethylamino, und wobei $R^2$ weiterhin auch Amino oder Dimethylamino bedeuten kann,

wobei von den Resten $R^1$ und $R^2$ mindestens einer ein chirales Zentrum enthalten muß,

$R^3$ und $R^4$, die gleich oder verschieden sein können, Wasserstoff oder $C_1$-$C_4$-Alkyl, das substituiert sein kann durch $C_1$-$C_4$-Alkoxy, Hydroxy, Fluor oder $C_1$-$C_4$-Dialkylamino,

das dadurch gekennzeichnet ist, daß man eine Verbindung der Formeln I oder II in Form ihres Racemats

mit einem Aminierungsreagenz zu einer Zwischenstufe, umsetzt, in der die in mindestens einem der Reste $R^1$, $R^2$, $R^3$ und $R^4$ enthaltene tertiäre Aminogruppe in eine Hydraziniumgruppierung der Formel III

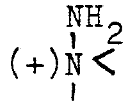 (III)

überführt wird, in der die durch Striche angedeuteten Bindungen zu Kohlenstoffatomen gehen, die auch Teil eines Ringsystems sein können, die Hydrazinium-Zwischenstufe in ihre Betainform überführt und diese durch Zugabe einer optisch aktiven, organischen Säure in diastereomere Salze überführt, die Enantiomeren durch Kristallisation dieser Salze trennt, die optisch aktive Säure wieder entfernt und schließlich die erhaltenen optisch aktiven Hydrazinium-Zwischenstufen durch reduktive Spaltung in die optisch aktiven Enantiomeren einer Verbindung der Formeln I oder II überführt.

Das folgende Reaktionsschema verdeutlicht die wesentlichen Schritte:

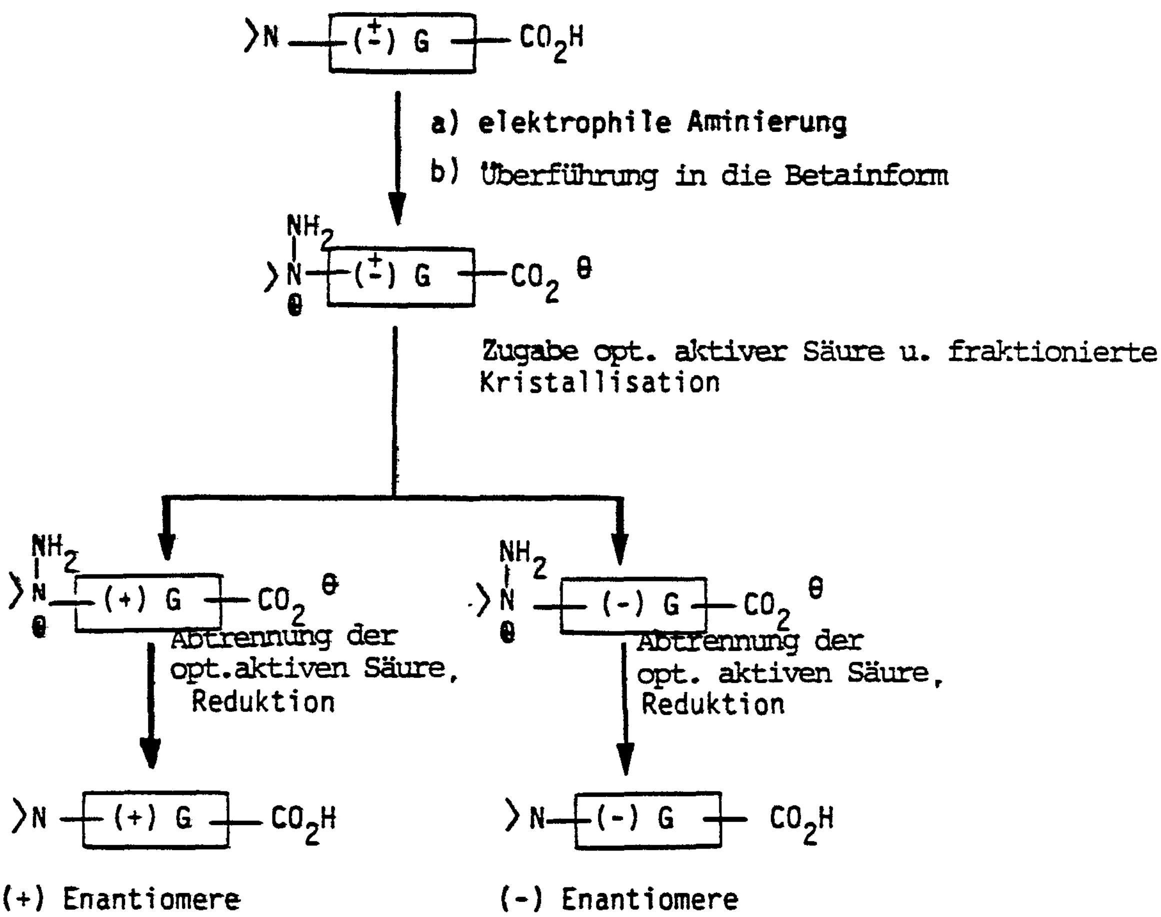


(±) G = Molekülgerüst des racemischen Gyrasehemmers

(+) G = Molekülgerüst des rechtsdrehenden Gyrasehemmers

(-) G = Molekülgerüst des linksdrehenden Gyrasehemmers

Die vorliegende Erfindung betrifft insbesondere die Herstellung optisch aktiver Verbindungen, in denen

die Substituenten in den Formeln I und II die folgenden Bedeutungen haben:
X = -CH=, -N= oder =CF-, vorzugsweise -CH=
Y = Wasserstoff oder Halogen, vorzugsweise Halogen, insbesondere Fluor,
Z = -CH=, -N=, =CF-und

$$\begin{array}{l} =C- \\ \ | \\ O-C_1-C_3\text{-Alkyl} \end{array} ,$$

wobei -CH= und =CF-bevorzugt sind und der Rest $C_1$-$C_3$-Alkyl insbesondere für Methyl stehen kann,
A = -$CH_2$-, -O-oder -S-, vorzugsweise -O-
$R^1$ = durch Dimethylamino oder Diethylamino substituiertes $C_1$-$C_4$-Alkyl, wobei der substituierte Alkylrest vorzugsweise für Methyl, Ethyl, Propyl oder Isopropyl stehen kann,
ein gesättigter 5-oder 6-gliedriger Ring mit 1 bis 2 Stickstoffatomen, wie insbesondere Pyrrolidinyl, Piperidinyl oder Piperazinyl, oder ein ungesättigter 5-oder 6-gliedriger Ring mit 1 bis 2 Stickstoffatomen, wie insbesondere Pyrrolyl, Imidazolyl oder Pyridyl, wobei die vorgenannten, insbesondere die gesättigten 5-oder 6-gliedrigen Ringsysteme auch noch substituiert sein können durch $C_1$-$C_4$-Alkyl, vorzugsweise Methyl, durch Hydroxy-$C_1$-$C_4$-Alkyl, vorzugsweise Hydroxymethyl oder durch $C_1$-$C_4$-Dialkylamino, vorzugsweise Dimethylamino, wobei als Beispiele für substituierte Ringe besonders interessant sind N-Methyl-piperazinyl, 2-Methyl-piperazinyl, 3-Methyl-pyrrolidinyl oder 2-Hydroxymethyl-pyrrolidinyl,
$R^2$ = $C_1$-$C_4$-Alkyl, vorzugsweise Methyl, Ethyl, Propyl, Isopropyl, insbesondere Ethyl,
$C_2$-$C_3$-Alkenyl, insbesondere Vinyl, Cyclopropyl, Aryl, vorzugsweise Phenyl, Amino und Dimethylamino, wobei die vorstehenden Reste $R^2$ (mit Ausnahme von Amino und Dimethylamino) auch substituiert sein können durch Chlor oder Fluor, wobei Fluor bevorzugt ist, Hydroxy, Methoxy, Ethoxy oder Diethylamino, wobei für die substituierten Reste besonders interessant sind Fluorethyl, vorzugsweise 2-Fluorethyl, Hydroxyethyl, vorzugsweise 2-Hydroxyethyl, Difluorethyl, Trifluorethyl, Methoxyethyl, Fluorcyclopropyl, Difluorcyclopropyl, 2-und 4-Fluorphenyl, 2,4-Difluorphenyl,
$R^3$ und $R^4$, die gleich oder verschieden sein können, können stehen für Wasserstoff, $C_1$-$C_4$-Alkyl, vorzugsweise Methyl, das auch noch weiter substituiert sein kann, durch $C_1$-$C_4$-Alkoxy, vorzugsweise Methoxy, durch Hydroxy, durch Fluor oder durch $C_1$-$C_4$-Dialkylamino, vorzugsweise Dimethylamino.

Erfindungsgemäß besonders interessant ist die Herstellung des therapeutisch sehr wichtigen, linksdrehenden Ofloxacins (Formel II mit Y = Fluor,

$R^1$ = 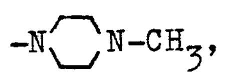 ,

$R^4$ = Wasserstoff, A = Sauerstoff, X = -CH= und $R^3$ = Methyl).

Als Aminierungsreagenz kommen beispielsweise in Betracht Chloramin T, insbesondere die Hydroxylamin-O-sulfonsäure, sowie auch deren Derivate, wie z.B. 0-Mesitylen-sulfonylhydroxylamin, oder das 0-(Diphenylphosphinyl)-hydroxylamin.

Zur Trennung in die Antipoden sind die hierfür literaturbekannten optisch aktiven organischen Säuren geeignet, beispielsweise die optisch aktiven Formen der Weinsäure, Apfelsäure, Campher-10-sulfonsäure, 3-Bromcampher-8-sulfonsäure, 3-Brom-campher-10-sulfonsäure, Dibenzoylweinsäure, Di-(4-toluoyl)-weinsäure, das Phenylethyl-bernsteinsäure-monoamid, vorzugsweise die optisch aktiven Formen der Mandelsäure.

Die racemischen Verbindungen der Formeln I and II sind literaturbekannt oder nach literaturbekannten Verfahren herstellbar.

Zur erfindungsgemäßen, elektrophilen Aminierung der tertiären Aminogruppe der Gyrasehemmer kann zweckmäßigerweise die im Handel erhältliche Hydroxylamin-0-sulfonsäure verwendet werden. Ebensogut lassen sich auch andere Aminierungsreagenzien, wie z.B. Chloramin T, oder beispielsweise die obengenannten 0-substituierten Hydroxylamine einsetzen.

Normalerweise ist es ausreichend, äquimolare Mengen oder einen geringen, beispielsweise bis zu 20 %igen Überschuß an Aminierungsreagenz einzusetzen.

Wasser ist, insbesondere bei Verwendung von Hydroxylamin-0-sulfonsäure, ein geeignetes Lösungsmittel für diesen Reaktionsschritt. Ein Zusatz von organischen Lösungsmitteln, wie z.B. Methanol, Ethanol, Isopropanol, Dimethylformamid, ist möglich, falls die Ausgangsstoffe in Wasser wenig löslich sind.

In der Regel wird die Umsetzung bei Raumtemperatur durchgeführt und erstreckt sich über einige

Stunden.

Die racemische Hydrazinium-Stufe wird aus den bei der Aminierung und der zweckmäßigerweise folgenden Ausfällung (z.B. als Hydrochlorid) entstandenen Salzen durch Behandlung mit einer basischen Komponente, beispielsweise mit einem üblichen basischen Ionenaustauscher, wie z.B. Serdolit, oder einer anorganischen Base, wie z.B. Natronlauge, in die Betainform überführt, die durch weitere Ausfällung als Salz, (beispielsweise das Hydrochlorid) und erneute Freisetzung des Betains noch weiter gereinigt werden kann.

Zur Trennung in die Antipoden über diastereoisomere Salze sind verschiedene, beispielsweise die oben erwähnten optisch aktiven Säuren brauchbar. Als besonders günstig hat sich (+)Mandelsäure und (-)Mandelsäure erwiesen, die zur rascheren Anreicherung der Enantiomeren auch im Wechsel eingesetzt werden können, d.h. man verwendet erst (+)Mandelsäure zur Anreicherung der (-)Form der Hydrazinium-Zwischenstufen und versetzt dann die Mutterlauge, die die (+)Form der Hydrazinium-Zwischenstufe im Überschuß enthält, mit der (-) Form der Mandelsäure. Die Trennung in die Antipoden erfolgt in an sich bekannter Weise und wird durch Verfolgen der optischen Drehung kontrolliert. In Abhängigkeit von der Löslichkeit der zur Trennung eingesetzten optisch aktiven Säuren sind die Lösungsmittelmengen entsprechend anzupassen.

Zur reduktiven Spaltung der optisch aktiven Hydrazinium-Zwischenstufen in die optisch aktiven Verbindungen der Formeln I und II eignen sich die üblichen Reduktionsverfahren, wie beispielsweise mit Zinkstaub, durch elektrolytische Reduktion, vorzugsweise jedoch katalytisch erregter Wasserstoff. Die Spaltung gelingt im allgemeinen bei Normaldruck, kann aber auch bei erhöhtem Druck durchgeführt werden, wenn eine weitere Beschleunigung der Hydrierung erwünscht wird. Als Katalysatoren eignen sich alle Hydrierungskatalysatoren, die keine Addukte bilden oder Reaktionen mit den Substraten eingehen. Als bevorzugte Katalysatoren werden die Edelmetall-Katalysatoren Palladium und Platin angesehen. Es genügt bereits der in der Literatur für derartige Hydrierungen beschriebene Einsatz sehr kleiner Katalysatormengen, die mehrmals verwendet werden können.

Das erfindungsgemäße Verfahren kann besonders vorteilhaft so durchgeführt werden, daß man zunächst zur elektrophilen Aminierung eine racemische Verbindung der Formeln I oder II, beispielsweise (±)Ofloxacin, in Wasser suspendiert und bei Raumtemperatur oder leicht erhöhter Temperatur durch Zugabe von Alkali, wie z.B. festem und/oder in Wasser gelöstem Natriumhydrogencarbonat oder Natriumcarbonat in Lösung bringt und eine, beispielsweise mit Natriumbicarbonat neutralisierte wässrige Lösung des Aminierungsreagenzes, vorzugsweise von Hydroxylamin-0-sulfonsäure, in äquimolarer Menge oder vorzugsweise einem etwa 10 %igen Überschuß hinzufügt. Nach längerem Stehen (beispielsweise von etwa 20 Stunden) bei Raumtemperatur wird angesäuert, beispielsweise mit 4n Salzsäure, bis auf einen pH-Wert von etwa 3, wobei das racemische, aminierte Produkt als Hydrochlorid ausfällt.

Die Enantiomerentrennung kann dann besonders vorteilhaft so durchgeführt werden, daß man das Hydrochlorid des racemischen Aminierungsproduktes bei leicht erhöhter Temperatur in Wasser löst, einen handelsüblichen, basischen Ionenaustauscher, wie z.B. Serdolit Blue® hinzufügt, eine gewisse Zeit, beispielsweise eine halbe Stunde, zweckmäßigerweise unter Rühren, stehen läßt, den Austauscher beispielsweise durch Filtration oder Abschleudern abtrennt und das Filtrat vorsichtig, vorzugsweise im Vakuum bei Raumtemperatur oder schwach erhöhter Temperatur eindampft. Man löst dann den Rückstand (Betain) in Wasser und fügt die wässrige Lösung der äquimolaren Menge einer optisch aktiven organischen Säure, beispielweise von (S)(+)-Mandelsäure, hinzu. Nach Einengen im Vakuum bei Raumtemperatur bis schwach erhöhter Temperatur und anschließendem mehrstündigem Stehen (beispielsweise etwa 20 Stunden) im Eisbad scheidet sich eines der beiden Enantiomeren der aminierten Zwischenstufe in Form eines diastereoisomeren Salzes ab. Das Salz muß dann im allgemeinen durch mehrmaliges (beispielsweise 5-bis 7-maliges) Umkristallisieren, beispielsweise aus Wasser, bis zur Konstanz des Drehwertes gereinigt werden.

Die Abtrennung und Rückgewinnung der optisch aktiven Säure erfolgt beispielsweise so, daß das erhaltene diastereomere Salz, beispielsweise mit der (S)(+)Mandelsäure, in Wasser gelöst und mit einem basischen Ionenaustauscher, wie beispielsweise Serdolit, einige Minuten (beispielsweise 15 Minuten) gerührt wird. Da andere Enantiomere der aminierten Zwischenstufe kann aus der Mutterlauge in derselben Weise isoliert werden, nur mit der Abweichung, daß beispielsweise die (R)(-)Mandelsäure zur Abscheidung des (+)Enantiomeren hinzugefügt wird.

Gegebenenfalls noch vorhandene Reste an nicht aufgetrenntem, racemischem Anteil der aminierten Zwischenstufe können dadurch zurückgewonnen werden, daß man die vereinigten Mutterlaugen aller Operationen beispielsweise mit Salzsäure auf einen pH-Wert von etwa 3 einstellt und so ihre Hydrochloride ausfällt.

Um die optisch aktiven Verbindungen der Formeln I und II, beispielsweise von Ofloxacin zu erhalten, muß noch die Aminogruppe (vgl. Formel III) abgespalten werden. Dies kann durch reduktive Desaminierung

beispielsweise so erfolgen, daß man die Lösung der linksdrehenden bzw. rechtsdrehenden aminierten Zwischenstufe beispielsweise mit Eisessig ansäuert und nach Zugabe eines Hydrierungskatalysators, beispielsweise von Palladium, bei Normaldruck in an sich bekannter Weise hydriert, was in der Regel etwa 2 bis 4 Stunden in Anspruch nimmt. Nach Absaugen vom Katalysator kann beispielsweise die Lösung durch Lyophilisieren zur Trockne gebracht, der Rückstand in einem geeigneten Lösungsmittel, wie z.B. in Ethanol, aufgenommen, durch Abkühlen zum Auskristallisieren gebracht und gegebenenfalls noch weiter umkristallisiert werden.

Das erfindungsgemäße Verfahren zeichnet sich dadurch aus, daß es fast ausschließlich in Wasser durchgeführt wird, ohne Verwendung teuerer Apparate und Chemikalien.

Da bisher für Gyrasehemmer keine brauchbaren Verfahren für die Spaltung in ihre optischen Antipoden beschrieben wurden, war es nicht zu erwarten, daß durch die Überführung in die Hydrazinium-Zwischenstufe eine Komponente entstehen würde, die sich aufgrund ihrer physikochemischen Eigenschaften besonders leicht in ihre Antipoden spalten und anschließend in den optisch aktiven Gyrasehemmer überführen läßt.

Gegenstand der Erfindung sind nicht nur das vorstehend beschriebene, neue Spaltungsverfahren, sondern auch die damit erhaltenen pharmazeutisch wirksamen, optisch aktiven Antipoden der Verbindungen I und II, die - mit Ausnahme von Ofloxacin -bisher nicht hergestellt werden konnten.

Gegenstand der Erfindung sind weiterhin auch die neuen, racemischen und optisch aktiven Hydrazinium-Zwischenverbindungen der Gyrasehemmer der Formeln I und II, in denen die tertiäre(n) Aminogruppe(n) als Hydrazinium-Gruppierung der Formel III

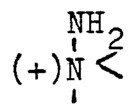

(III)

vorliegt. Sie stellen die entscheidenden Zwischenprodukte für die erfindungsgemäße Spaltung in die optischen Antipoden dar.

Die pharmazeutisch wirksamen, optisch aktiven Antipoden der Verbindungen der Formeln I und II, sind wertvolle Antibiotika und können in reiner Form mit den üblichen galenischen Hilfsstoffen oder auch in Kombination mit anderen Antibiotika oral und parenteral in der Human-und Veterinär-Medizin angewendet werden. Arzneimittel können so hergestellt werden, daß man eine pharmazeutisch wirksame, optisch aktive Antipode der Verbindungen der Formeln I oder II mit einem oder mehreren pharmakologisch verträglichen Trägerstoffen oder Verdünnungsmitteln, wie z.B. Füllstoffen, Emulgatoren, Gleitstoffen, Geschmackskorrigentien oder Puffersubstanzen vermischt und in eine geeignete galenische Zubereitungsform bringt, wie beispielsweise Tabletten, Dragees, Kapseln, oder eine zur parenteralen Applikation geeignete Suspension oder Lösung.

Als Träger-oder Verdünnungsmittel seien beispielsweise erwähnt Traganth, Milchzucker, Talkum, Agar-Agar, Polyglykole, Ethanol und Wasser. Für die parenterale Applikation kommen vorzugsweise Suspensionen oder Lösungen in Wasser in Betracht. Es ist auch möglich, die Wirkstoffe als solche ohne Träger-oder Verdünnungsmittel in geeigneter Form, beispielsweise in Kapseln, zu applizieren. Geeignete Dosen der Wirkstoffe liegen bei etwa 0,05 bis 1 g/Tag, vorzugsweise bei etwa 0,05 bis 0,5 g/Tag.

Es können Einzel-oder im allgemeinen Mehrfachdosen verabreicht werden, wobei die Einzeldosis den Wirkstoff in der vorstehend angegebenen Menge enthalten kann.

Zur Erläuterung der Erfindung wird als typisches Beispiel die Herstellung der zur Enantiomerentrennung erforderlichen Hydrazinium-Schlüsselverbindung von (±)Ofloxacin beschrieben, ihre Spaltung in die Antipoden sowie deren Reduktion zu den optisch aktiven Enantiomeren.

<u>Beispiel: Trennung von (±)-9-Fluor-2,3-dihydro-3-methyl-10-(4-methyl-1-piperazinyl)-7-oxo-7H-pyrido [1,23-de]-[1.4.]-benzoxazin-6-carbonsäure in die optisch aktiven Enantiomeren</u>

A. <u>Aminierung mit Hydroxylamin-0-sulfonsäure</u>

216.8 g (0.6 Mol) (±)-9-Fluor-2,3-dihydro-3-methyl-10-(4-methyl-1-piperazinyl)-7-oxo-7H-pyrido[1,2,3-de]-[1.4]-benzoxazin-6-carbonsäure suspendiert man in 3 l Wasser und setzt bei Raumtemperatur unter Rühren 101 g festes Natriumhydrogenkarbonat und 1,2 1 2n-Natriumkarbonatlösung zu. Es entsteht rasch eine klare Lösung, zu der man eine neutralisierte Lösung von Hydroxylamin-0-Sulfonsäure, hergestellt aus 75 g (0.66 Mol) Hydroxylamin-0-Sulfonsäure, der äquivalenten Menge Natriumhydrogenkarbonat und 600 ml Wasser,

unter Rühren zugibt. Die Reaktionslösung wird nach 20 h Stehen bei Raumtemperatur unter Eiskühlung mit 4 n Salzsäure (ca. 900 ml) auf einen pH-Wert von ca. 3 gestellt. Das aminierte Produkt fällt sofort als Hydrochlorid kristallin aus, wird auf einer Nutsche abgesaugt und viermal mit je 100 ml Eiswasser ausgewaschen.

Analyse $C_{18}H_{21}FN_4O_4$ x HCl, $H_2O$-Gehalt 3 %
C ber. 50,8 % gef. 50,8 %
H ber. 5,5 % gef. 5,4 %
N ber. 13,2 % gef. 13,0 %

B. Enantiomerentrennung mit optisch aktiver Mandelsäure

Die Enantiomerentrennung kann mit dem noch feuchten Niederschlag durchgeführt werden. Zu diesem Zweck löst man den Niederschlag in 8 l Wasser bei 45°C auf, fügt 700 g eines stark basischen Austauschers zu [Serdolit Blue® (Serva), Korngröße 0.3 -0.9 nm] und rührt ca. eine halbe Stunde. Danach wird der Austauscher durch Absaugen oder Abschleudern entfernt und zweimal mit 500 ml Wasser gewaschen. Das Waschwasser wird mit dem Filtrat vereinigt und die ganze Flüssigkeitsmenge im Vakuum (ca. 20 mm) bei 20 - 40°C Badtemperatur abdestilliert. Den kristallinen Rückstand, der noch geringe Mengen Wasser enthält, löst man bei 40°C in 1.1 l Wasser und setzt eine bei 40° hergestellte Lösung von 91.3 g (0.6 Mol) (S) (+)-Mandelsäure in 400 ml Wasser zu. Die klare Lösung wird bei Wasserstrahlvakuum und 20 - 40° Badtemperature auf 1 l Gesamtvolumen eingeengt und ca. 20 Stdn. im Eisbad gekühlt. Nur das (-)-Enantiomere der aminierten Zwischenstufe scheidet sich als Salz der (S) (+)-Mandelsäure ab und wird nach dem Absaugen zweimal mit je 50 ml Eiswasser geswaschen und getrocknet. Zur Reinigung dieses Rohprodukts wird wie folgt verfahren:

Man kristallisiert das Rohprodukt fünf-bis siebenmal aus je 150 ml Wasser durch Erhitzen auf ca. 75° und anschließendes Kühlen im Eisbad um. Der Drehwert zeigt keinen weiteren Anstieg. Nach dem Trocknen über Phosphorpentoxyd im Vakuum erhält man 126 g von Zers. P. 239 - 241°.

Analyse $C_{18}H_{21}FN_4O_4$ x $C_8H_8O_3$ (528.6), $H_2O$-Gehalt: 2 %
C ber. 57.9 % gef. 58.0 %
H ber. 5.6 % gef. 5.4 %
N ber. 10.4 % gef. 10.4 %

Zur Bestimmung der optischen Reinheit werden 0.2 g in 30 ml Wasser gelöst, mit 2 g Serdolit-Austauscher 15 Min. bei Raumtemperatur gerührt, der Austauscher abgesaugt und das Filtrat einrotiert. Von den über $P_2O_5$ getrockneten und anschließend an der Luft rehydratisierten Kristallen wurde die optische Drehung bestimmt $[\alpha]_D^{26}$ = - 61° ± 1 (C = 1.0 in $H_2O$). Das zweite (+)-Enantiomere der aminierten Zwischenstufe befindet sich in der Mutterlauge und wird wie folgt isoliert:

Man setzt 350 g Serdolit dieser Mutterlauge zu, rührt 30 Minuten bei Raumtemperatur, saugt den Austauscher ab und wäscht dreimal mit je 200 ml Wasser nach. Die vereinigten Filtrate versetzt man mit 64 g (0.42 Mol) (R)(-)-Mandelsäure, engt auf 800 ml ein und kühlt ca. 20 Stdn. im Eisbad. Das (+)-Enantiomere der aminierten Zwischenstufe kristallisiert als Salz der (R)(-)-Mandelsäure aus und wird nach dem Absaugen zweimal mit je 50 ml Eiswasser gewaschen. Die Reinigung und Reinheitsbestimmung erfolgt analog der für das (-)-Enantiomere oben beschriebenen Vorschrift. Man erhält 99.2 g vom Zers. P. 239 -241°. $[\alpha]_D^{26}$ = + 60° ± 1° (C = 1.0 in $H_2O$)

Analyse $C_{18}H_{21}FN_4O_4$ x $C_8H_8O_3$ (528.6), $H_2O$-Gehalt: 3 %
C ber. 57.3 % gef. 57.0 %
H ber. 5.7 % gef. 5.3 %
N ber. 10.28 % gef. 10.1 %

Die gesammelten Mutterlaugen aller Operationen werden mit Salzsäure auf pH = 3 eingestellt, wobei der nicht aufgetrennte racemische Anteil der aminierten Zwischenstufe (59 g = 22.3 %) als Hydrochlorid zurückgewonnen wird.

C. Reduktive Desaminierung

42.6 g der aminierten Zwischenstufe vom Zers. P. 239 - 241°,$[\alpha]_D^{26}$ = -61° löst man in 1.2 l wasser bei 40°, setzt 112 g Serdolit-Austauscher zu, rührt 15 Min., saugt den Austauscher ab und wäscht zweimal mit je 100 ml Wasser nach. Das Filtrat wird mit 4.6 ml Eisessig angesäuert und nach Zugabe katalytischer Mengen Palladium bei Normaldruck unter Schütteln hydriert. Die berechnete Menge Wasserstoff wird

innerhalb von 2 bis 4 Stdn. Aufgenommen. Die Hydrierzeit hängt von der Menge des Katalysators ab. Man saugt vom Katalysator ab, wäscht mit wenig Wasser nach (ca. 50 ml) und lyophilisiert die Lösung. Der Rückstand wird in 400 ml Äthanol eine Stunde im Eisbad gerührt, die Kristalle abgesaugt, zweimal mit 40 ml kaltem Äthanol gewaschen und das noch feuchte Produkt aus 320 ml Äthanol umkristallisiert. Nach zweimaligem Waschen mit je 25 ml Äthanol erhält man 22,3 g (-)-9-Fluor-2,3-dihydro-3-methyl-10-(4-methyl-1-piperazinyl)-7-oxo-7H-pyrido[1.2.3-de]-[1.4]-benzoxazin-6-carbonsäure vom Zers. P. 218 - 219° $[\alpha]^{23}_{D}$ = -76.4 ± 1° (C = 0.385 in 0.05 n NaOH).

Anaylse $C_{18}H_{20}FN_3O_4$ (361,38), $H_2O$-Gehalt 1%

C ber. 59,2 % gef. 59,2 %

H ber. 5,6 % gef. 5,6 %

N ber. 11,5 % gef. 11,5 %

Das (+)-Enantiomere erhält man in analoger Weise ausgehend von der beschriebenen rechtsdrehenden Zwischenstufe vom Zers. P. 239 - 241°, $[\alpha]^{26}_{D}$ = +60 ± 1°.

Die Ausbeute an (+)-9-Fluor-2,3-dihydro-3-methyl-10-(4-methyl-1-piperazinyl)-7-oxo-7H-pyrido[1.2.3-de]-[1.4]-benzoxazin-6-carbonsäure beträgt 23,45 g, Zers. P. 218 - 219° $[\alpha]^{23}_{D}$ = +76.6 ± 1° (C = 0.385 in 0.05 n NaOH).

Analyse $C_{18}H_{20}FN_3O_4$ (361,38), $H_2O$-Gehalt 2 %

C ber. 58,6 % gef. 58,5 %

H ber. 5,7 % gef. 5,7 %

N ber. 11,4 % gef. 11,5 %

## Ansprüche

1. Verfahren zur Herstellung der optisch aktiven Antipoden von Gyrasehemmern der allgemeinen Formeln I und II

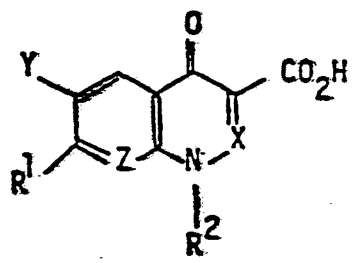

(I)

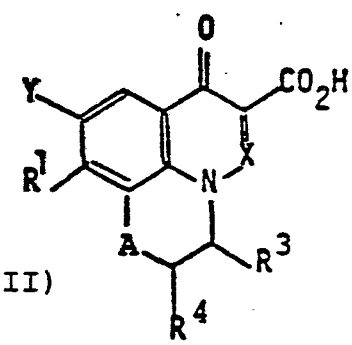

(II)

in denen bedeuten

X = -CH=, -N= oder =CF-

Y = Wasserstoff oder Halogen,

Z = -CH=, -N=, =CF-oder

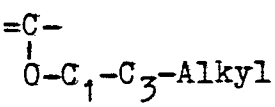

A = $-CH_2-$, -O-oder -S-

$R^1$ = $C_1$-$C_4$-Alkyl, das durch Dimethylamino oder Diethylamino substituiert ist, gesättigter oder ungesättigter 5-oder 6-gliedriger Ring mit 1 bis 2 Stickstoffatomen, der auch weiter substituiert sein kann durch $C_1$-$C_4$-Alkyl, Hydroxy-$C_1$-$C_4$-alkyl oder $C_1$-$C_4$-Dialkylamino,

$R^2$ = $C_1$-$C_4$-Alkyl, $C_2$-$C_3$-Alkenyl oder Cyclopropyl, wobei diese Reste auch substituiert sein können durch Fluor, Chlor, Hydroxy, Methoxy, Ethoxy oder Diethylamino, und wobei $R^2$ weiterhin auch Amino oder Dimethylamino bedeuten kann,

wobei von den Resten $R^1$ und $R^2$ mindestens einer ein chirales Zentrum enthalten muß,

$R^3$ und $R^4$, die gleich oder verschieden sein können, Wasserstoff oder $C_1$-$C_4$-Alkyl, das substituiert sein kann durch $C_1$-$C_4$-Alkoxy, Hydroxy, Fluor oder $C_1$-$C_4$-Dialkylamino

dadurch gekennzeichnet, daß man eine Verbindung der Formeln I oder II in Form ihres Racemats mit einem Aminierungsreagenz zu einer Zwischenstufe umsetzt, in der die in mindestens einem der Reste $R^1$, $R^2$, $R^3$ und $R^4$ enthaltene tertiäre Aminogruppe in eine Hydraziniumgruppierung der Formel III

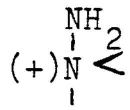 (III)

überführt wird, in der die durch Striche angedeuteten Bindungen zu Kohlenstoffatomen gehen, die auch Teil eines Ringsystems sein können, die Hydrazinium-Zwischenstufe in ihre Betainform überführt und diese durch Zugabe einer optisch aktiven, organischen Säure in diastereomere Salze überführt, die Enantiomeren durch Kristallisation dieser Salze trennt, die optisch aktive Säure wieder entfernt und schließlich die erhaltenen optisch aktiven Hydrazinium-Zwischenstufen durch reduktive Spaltung in die optisch aktiven Enantiomeren einer Verbindung der Formeln I oder II überführt.

2. Verfahren gemäß Anspruch 1, dadurch gekennzeichnet, daß als Aminierungsreagenz Hydroxylamin-O-sulfonsäure eingesetzt wird.

3. Verfahren gemäß Ansprüchen 1 und 2, dadurch gekennzeichnet, daß als optisch aktive Säure rechtsdrehende oder linksdrehende Mandelsäure eingesetzt wird.

4. Verfahren gemäß Ansprüchen 1 - 3, dadurch gekennzeichnet, daß ein Racemat der Verbindung, II eingesetzt wird, in der X für -CH=, A für Sauerstoff, Y für Fluor,

$R^1$ für 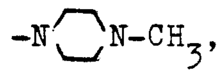

, $R^3$ für methyl und $R^4$ für Wasserstoff steht (Ofloxacin).

5. Die pharmazeutisch wirksamen, optisch aktiven Antipoden der in Anspruch 1 genannten Verbindungen der Formeln I und II, wobei eine Verbindung der Formel II ausgenommen ist, in der X für -CH=, A für Sauerstoff, Y für Fluor,

$R^1$ für 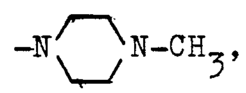

$R^3$ für Methyl und $R^4$ für Wasserstoff steht (Ofloxacin).

6. Racemate und optisch aktive Antipoden der Hydrazinium-Verbindungen der allgemeinen Formeln I und II, in denen eine enthaltene tertiäre Aminogruppe in eine Hydraziniumgruppierung der Formel III

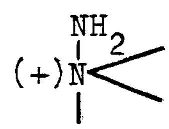 (III)

überführt wurde, in der die durch Striche angedeuteten Bindungen zu Kohlenstoffatomen gehen, die auch Teil eines Ringsystems sein können.

7. Verbindungen gemäß Anspruch 6, denen eine Verbindung der Formel II zugrundeliegt, in der X für -CH=, A für Sauerstoff, Y für Fluor,

$R^1$ für 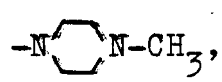

$R^3$ für Methyl und $R^4$ für Wasserstoff steht.

8. Gegen bakterielle Infektionen wirksame pharmazeutische Präparate, gekennzeichnet durch einen Gehalt an einem pharmazeutisch wirksamen, optisch aktiven Antipoden der Verbindungen der Formeln I und II.

9. Verfahren zur Herstellung von gegen bakterielle Infektionen wirksamen pharmazeutischen Präparaten, dadurch gekennzeichnet, daß ein pharmazeutisch wirksamer, optisch aktiver Antipode der Verbindungen der Formeln I und II gegebenenfalls mit pharmazeutisch üblichen Trägerstoffen in eine pharmazeutisch geeignete Verabreichungsform gebracht wird.

10. Verwendung von pharmazeutisch wirksamen, optisch aktiven Antipoden der allgemeinen Formeln I und II zur Bekämpfung bakterieller Infektionen.